# EUROPÄISCHE PATENTSCHRIFT

(11) **EP 2 822 993 B1**
(45) Veröffentlichungstag und Bekanntmachung des Hinweises auf die Patenterteilung: **17.02.2016**
(21) Anmeldenummer: 13706566.0
(22) Anmeldetag: 01.03.2013
(51) Int. Cl.: C08K 5/107, C07C 67/62, C08L 33/14

(54) **STABILISIERTE (METH)ACRYLMONOMERE**
STABILISED (METH)ACRYLIC MONOMERS
MONOMÈRES (METH)ACRYLIQUES STABILISÉS

(30) Priorität: 05.03.2012 DE 102012203362
(43) Veröffentlichungstag der Anmeldung: 14.01.2015
(73) Patentinhaber: Evonik Röhm GmbH, 64293 Darmstadt (DE)
(72) Erfinder: BREINER, Christine Maria, 69514 Laudenbach (DE); DORN, Klaus, 63457 Hanau (DE); KNEBEL, Joachim, 64665 Alsbach-Hähnlein (DE); SCHÜTZ, Thorben, 64665 Alsbach-Hähnlein (DE)
(86) Internationale Anmeldenummer: PCT/EP2013/054159
(87) Internationale Veröffentlichungsnummer: WO 2013/131818

(56) Entgegenhaltungen:
- EP-A1- 0 620 206
- EP-A2- 1 125 919
- WO-A1-03/006417

## Beschreibung

Die vorliegende Erfindung betrifft die Stabilisierung von (Meth)acrylmonomeren gegen vorzeitige Polymerisation während Synthese, Lagerung und Transport. Unter der Bezeichnung (Meth)acrylmonomere werden sowohl Derivate der Methacrylsäure als auch der Acrylsäure verstanden.

Aufgrund der Polymerisationsneigung von (Meth)acrylverbindungen ist es allgemein üblich, diese gegen vorzeitige Polymerisation zu stabilisieren und sie während Synthese, Lagerung und Transport mit Polymerisationsinhibitoren zu versetzten. Als Polymerisationsinhibitoren wurden bis dato zahlreiche Verbindungen eingesetzt, einschließlich Phenolverbindungen, wie z.B. Hydrochinon, Methylhydrochinon, tert.-Butylhydrochinon, 2,6-Di-tert.-butyl-para-hydrochinon, 2,5-Di-tert.-butyl-hydrochinon, 2,4-Dimethyl-6-tert.-butyl-phenol und Hydrochinon-monomethylether, oder para-Phenylendiamine wie z.B. N-Isopropyl-N'-phenyl-para-phenylendiamin, N-(1,3-Dimethlbutyl)-N'-phenyl-para-phenylendiamin, N-(1-Methylheptyl)- N'-phenyl-para-phenylendiamin, N,N'-Diphenyl-para-phenylendiamin und N,N'-Di-2-naphthyl-para-phenylendiamin oder Amine wie z.B. Thiodiphenylamin.

Beispielsweise beschreibt die EP 0522709 bestimmte N,N'-Dinitroso-phenylendiamine zur Stabilisierung von Acrylsäureestern. Ferner werden in EP 0620206 Stabilisatorkombinationen aus mindestens einer N-Oxyl-Verbinung, mindestens einer Phenol-Verbindung und mindestens einem Phenothiazin beansprucht.

Weitere Stabilisatoren sind in Römpp-Lexikon Chemie; Herausgeber: J. Falbe, M. Regitz; Stuttgart, New York; 10. Auflage (1996); Stichwort "Antioxidantien" und den an dieser Stelle zitierten Literaturstellen beschrieben.

Trotz dieser Vielzahl von bereits angebotenen Stabilisatorlösungen besteht für bestimmte höher reaktive (Meth)acrylmonomere, wie. z.B. Hydroxyethyl(meth)acrlat, nach wie vor Bedarf, eine effizientere Polymerisationsinhibierung, bzw. eine solche, die weniger Nebeneffekte, wie z.B. unerwünschte Verfärbungen verursacht, während Synthese, Transport und Lagerung zu realisieren.

Gelöst wird diese Aufgabe im Rahmen der vorliegenden Erfindung durch stabilisierte (Meth)acrylmonmere, die als Polymerisationsinhibitoren die Verbindung der Formel (1) enthalten.

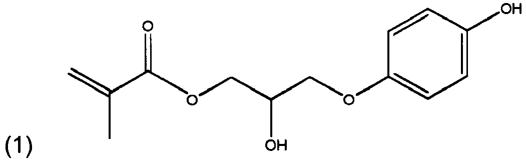

Die Anwesenheit dieser Verbindung als Polymerisationsinhibitor während Synthese, Lagerung und Transport von (Meth)acrylsäureestern gewährleistet eine ausreichende Stabilisierung ohne zu Verfärbungen zu führen. Ferner wird sie in der eigentlichen Anwendung dieser (Meth)acrylsäurester-Monomere zur Herstellung von Polymeren bei der Polymerisation aufgrund der vorhandenen Methacrylat-Funktion mit in die Polymerkette eingebaut, was z.B. ein unerwünschtes späteres Ausbluten (die Abgabe niedermolekularer Bestandteile) des Polymerisationsinhibitors verhindert.

Zur Herstellung der Verbindung der Formel (1) können dem Fachmann bekannte Methoden der synthetischen Organischen Chemie eingesetzt werden. Besonders geeignet ist die Herstellung aus Glycidylmethacrylat und Hydrochinon.

Geeignete Konzentrationen zum Erreichen einer Stabilisierungswirkung liegen bei 0,0001 bis 2 Gew%, bevorzugt bei 0,001 bis 1,5 Gew%, besonders bevorzugt bei 0,002 bis 1 Gew%, bezogen auf das zu stabilisierende (Meth)acrylmonomer, bzw. wenn Mischungen von (Meth)acrylmonomeren vorliegen, auf die Gesamtmenge der vorhandenen Monomere.

Zur Stabilisierung geeignete Monomere sind unter anderem (Meth)acrylsäureester, die sich von gesättigten Alkoholen ableiten, wie Hexyl(meth)acrylat, 2-Ethylhexyl(meth)acrylat, Heptyl(meth)acrylat, 2-(tert.-Butylamino)ethyl(meth)acrylat, Octyl(meth)acrylat, 3-iso-Propylheptyl(meth)acrylat, Nonyl(meth)acrylat, Decyl(meth)acrylat, Undecyl(meth)acrylat, 5-Methylundecyl(meth)acrylat, Dodecyl(meth)acrylat, 2-Methyldodecyl(meth)acrylat, Tridecyl(meth)acrylat, 5-Methyltridecyl(meth)acrylat, Tetradecyl(meth)acrylat, Pentadecyl(meth)acrylat, Hexadecyl(meth)acrylat, 2-Methylhexadecyl(meth)acrylat, Heptadecyl(meth)acrylat, 5-iso-Propylheptadecyl(meth)acrylat, 4-tert.-Butyloctadecyl(meth)acrylat, 5-Ethyloctadecyl(meth)acrylat, 3-iso-Propyloctadecyl(meth)acrylat, Octadecyl(meth)acrylat, Nonadecyl(meth)acrylat, Eicosyl(meth)acrylat, Cetyleicosyl(meth)acrylat, Stearyleicosyl(meth)acrylat, Docosyl(meth)acrylat und/oder Eicosyltetratriacontyl - (meth)acrylat; (Meth)acrylate, die sich von ungesättigten Alkoholen ableiten, wie z. B. 2-Propinyl(meth)acrylat, Allyl(meth)acrylat, Vinyl(meth)acrylat, Oleyl(meth)acrylat; Cycloalkyl(meth)acrylate, wie Cyclopentyl(meth)acrylat, 3-Vinylcyclohexyl(meth)acrylat, Cyclohexyl(meth)acrylat, Bornyl(meth)acrylat; (Meth)acrylate, die zwei oder mehr (Meth)acryl-Gruppen aufweisen, Glycoldi(meth)acrylate, wie Ethylenglycoldi(meth)acrylat, Diethylenglycoldi(meth)acrylat, Triethylenglycoldi(meth)acrylat, Tetra- und Polyethylenglycoldi(meth)acrylat, 1,3-Butandiol(meth)acrylat, 1,4-Butandiol(meth)acrylat, 1,6-Hexandioldi(meth)acrylat, Glycerindi(meth)acrylat und Dimethacrylate von ethoxyliertem Bisphenol A; (Meth)acrylate mit drei oder mehr Doppelbindungen, wie z.B. Glycerintri(meth)acrylat, Trimethylolpropantri(meth)acrylat, Pentaerythrittetra(meth)acrylat und Dipentaerythritpenta(meth)acrylat. Darüber hinaus gehören hierzu (Meth)acrylate, die sich von Polymeren mit mindestens einer Hydroxygruppe ableiten, wie zum Beispiel Polyalkylenglykolmono(meth)acrylate und Polyalkylenglykoldi(meth)acrylate. Ebenfalls zu den bevorzugten (Meth)acrylsäureestern gehören unter anderem Polyalkylenglykolmono(meth)acrylate, insbesondere Polyethylenglykolmono(meth)acrylate, Polypropylenglykolmono(meth)acrylate und Polybutylenglykolmono(meth)acrylate und Polyalkylenglykoldi(meth)acrylate, insbesondere Polyethylenglykoldi(meth)acrylate, Polypropylenglykoldi(meth)acrylate und Polybutylenglykoldi(meth)acrylate.

Zur erfindungsgemäßen Stabilisierung sind auch Hydroxyalkyl(meth)arylate geeignet. Bevorzugte Hydroxyalkyl(meth)acrylate umfassen u.a. 2-Hydroxyethyl(meth)acrylat, 2-Hydroxypropyl(meth)acrylat, 3-Hydroxypropyl(meth)acrylat und 3 ,4-Dihydroxybutyl(meth)acrylat. Von besonderem Interesse sind Verbindungen ausgewählt aus der Gruppe bestehend aus Hydroxyethylacrylat (HEA), Hydroxyethylmethacrylat (HEMA), 2-Hydroxypropylacrylat (2-HPA), 2-Hydroxypropylmethacrylat (2-HPMA), 3-Hydroxypropylacrylat (3-HPA), 3-Hydroxypropylmethacrylat (3-HPMA), 2 ,3-Dihydroxypropylacrylat, 2 ,3-Dihydroxypropyl methacrylat, 1,3-Diacryloyl-glycerin, 1,3-Dimethacryloyl-glycerin, Trimethylolpropanmonoacrylat, Trimethylolpropanmonomethacrylat, Trimethylolpropandiacrylat und Trimethylolpropandimethacrylat.

Die ethylenisch ungesättigten Monomere können einzeln oder als Mischung in der erfindungsgemäßen Zusammensetzung vorhanden sein.

Ferner geeignet zur Stabilisierung sind (Meth)acrylamide der Formel (2)

(2) CH₂=CR₃-CO-NHR²

wobei R³ Wasserstoff oder die Methylgruppe bedeutet. R² steht für einen linearen, verzweigten oder cyclischen Alkylrest, einen Arylrest, der auch mit ein oder mehreren Alkylgruppen substituiert sein kann. Der lineare, cyclische oder verzweigte Alkylrest kann eine Länge von 1 bis 12 Kohlenstoffatomen aufweisen, und beispielsweise Methyl, Ethyl, Propyl, Isopropyl, Butyl, Isobutyl, tert.-Butyl, Pentyl, Hexyl, Heptyl, Octyl, Isooctyl, Nonyl, Decyl, Undecyl bedeuten und kann gegebenenfalls durch -NR³R⁴ oder -OR⁵ein- oder mehrfach substituiert sein, dabei kann entweder R³ oder R⁴ die Bedeutung von Wasserstoff annehmen und wobei ferner gilt: R³, R⁴ oder R⁵ können entweder gleich oder verschieden sein und eine Alkylgruppe mit 1 bis 12 Kohlenstoffatomen darstellen, wie beispielsweise Methyl, Ethyl, Propyl, Isopropyl, Butyl, Isobutyl, tert. Butyl, Pentyl, Hexyl, Heptyl, Octyl, Isooctyl, Nonyl, Decyl, Undecyl oder Wasserstoff. R² kann ferner auch [(R⁶-O)ₙ]-R⁷ bedeuten, wobei gilt: n kann die Werte von 1 bis 4 annehmen; -R⁶ kann eine C1-C4-Alkylgruppe sein, die auch verzweigt sein kann, wie beispielsweise Methyl, Ethyl, Propyl, Isopropyl, Butyl, Isobutyl oder tert.-Butyl; R⁷ kann die Methylgruppe oder die Ethylgruppe sein

Besonders zur Stabilisierung geeignet sind (Meth)acrylatderivate basierend auf mehrfach ungesättigten Fettsäurekomponenten wie sie z.B. in EP 2217629, EP 2283075, EP2334634, DE 102009001964 und DE102009001965 beschrieben sind. Der Inhalt dieser Druckschriften gilt hiermit als offenbart. Hierbei handelt es sich im Wesentlichen um (Meth)acrylmonomere der Formel (3) 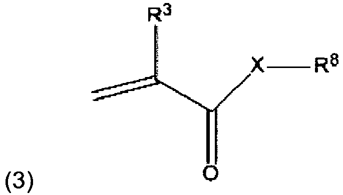 worin der Rest R³ Wasserstoff oder Methyl darstellt, -X- unabhängig 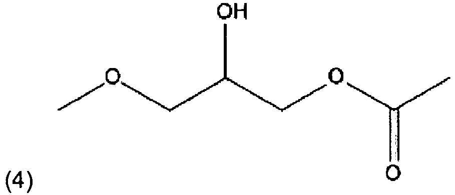 oder 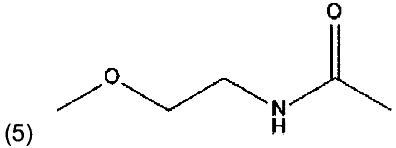 oder 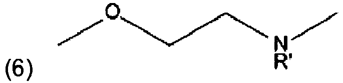 sein kann, worin R' Wasserstoff oder ein Rest mit 1 bis 6 Kohlenstoffatomen ist, und R⁸ einen linearen oder verzweigten Rest mit 8 bis 40, vorzugsweise 10 bis 30 und besonders bevorzugt 12 bis 20 Kohlenstoffatomen bedeutet, der mindestens eine C-C-Doppelbindung aufweist.

Die folgenden Beispiele sollen die Erfindung erläutern, aber in keiner Weise einschränken.

### Beispiel 1:

### Synthese des Glycidylmethacrylat-Hydrochinon Adduktes (Formel (1)):

Eine Mischung aus 9,95g Glycidylmethacrylat, 4,40g Hydrochinon, 0,050g HEPTCR 050 NPG (Chromheptanoat in Polypropylenglykol) und 50 ml o-Xylol wird in einen 250ml Vierhalskolben mit Säbelrührer (Rührhülse, Rührmotor), einem Thermometer, einem Einleitungsrohr für Pressluft, einem Rückflusskühler und einem Ölbad mit Temperaturregelung vorgelegt. Das Reaktionsgemisch wurde unter Lufteinleitung zum Sieden erhitzt. Anschließend wurde für 10h bei einer Reaktionstemperatur (=Kolbeninnentemperatur) von 142°C refluxiert. Nach Abkühlen auf Raumtemperatur wurden im Rotationsverdampfer (Badtemperatur 55°C, Vakuum 20mbara) die volatilen Bestandteile entfernt. Nach Filtration wurde eine braune, trübe Flüssigkeit erhalten. Das Syntheseprodukt wurde mittels Säulen- und Dünnschichtchromatographie aufgereinigt. Als stationäre Phase der Säulenchromatographie wurde Kieselgel 60 (0,063 - 0,200 mm) eingesetzt, das Laufmittel bestand aus einer Mischung Ethylacetat/Cyclohexan im Verhältnis 1/1. Die erhaltenen klaren und farblosen Lösungen wurden zur drei Fraktionen zusammengeführt. Das Laufmittel wurde anschließend im Rotationsverdampfer entfernt.

### Beispiel 2:

Die in Beispiel 1 hergestellte erfindungsgemäße Verbindung wurde in UHP HEMA (ultra high purity Hydroxyethylmethacrylat, kommerziell erhältlich als Visiomer^{®} UHP HEMA, bei Evonik Industries AG, Deutschland) eingebracht und deren Polymerisationszeit sowie Verfärbung via Farbzahl bestimmt. Die Ergebnisse sind in Tab. 1 dargestellt.

### Vergleichsbeispiel:

Als Vergleichsinhibitor wurde N,N-Diphenylendiamin in ultra high purity Hydroxyethylmethacrylat eingebracht und Polymerisationszeit sowie Verfärbung via Farbzahl bestimmt. Die Ergebnisse sind ebenfalls in Tab. 1 dargestellt.

**Tab. 1: Polymerisationszeiten und Farbzahlen**

| | Beispiel 2 | | Vergleichsbeispiel | |
|---|---|---|---|---|
| Konzentration / ppm | Polymerisationszeit / min | Farbzahl Pt/Co | Polymerisationszeit / min | Farbzahl Pt/Co |
| 0 | 70,7 | 8 | 71,2 | 8 |
| 5 | 70,5 | 12 | 102,7 | 59 |
| 20 | 75,0 | 18 | 183,3 | 193 |
| 50 | 81,3 | 28 | 325,8 | 400 |
| 100 | 94,8 | 35 | 589,2 | 676 |

Aus Tab. 1 wird klar ersichtlich, dass der erfindungsgemäße Inhibitor bei ausreichender Inhibitorleistung wesentlich bessere Farbwerte aufweist.

### Methode zur Bestimmung der Polymerisationszeit

Die Polymerisationszeit ist definiert als die Zeit, die ein Ansatz vom Polymerisationsstart bis zum Erreichen der Polymerisationsspitzentemperaturunter Zusatz eines Initiators benötigt. In den vorliegenden Beispielen wurden 0,1 % AlBN bei einer Polymerisationstemperatur von 55 °C eingesetzt. Als Ergebnis wird die benötigte Zeit angegeben. Die Messung erfolgt mittels Kontaktthermometer unter Aufzeichnung des Temperaturverlaufs.

### Methode zur Bestimmung der Farbzahl

Die Farbzahl wurde mittels des in DE 10131479 ausführlich dargelegten Verfahrens (Bestimmung der Farbe nach der Platin-Cobalt-Skala; auch APHA oder Trübungszahl genannt) bestimmt. Dieses Verfahren wurde in Anlehnung an DIN EN ISO 6271 entwickelt.

## Patentansprüche

1. Stabilisiertes (Meth)acrylmonomer, **dadurch gekennzeichnet, dass** es während Synthese, Lagerung und Transport als Polymerisationsinhibitor die Verbindung der Formel (1) 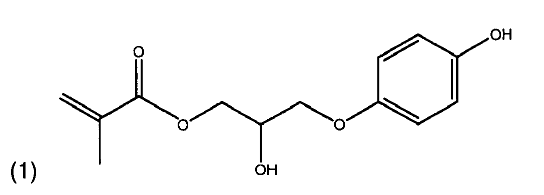 enthält.

2. Stabilisiertes (Meth)acrylmonomer nach Anspruch 1, **dadurch gekennzeichnet, dass** die Konzentration von (1) bei 0,0001 bis 2 Gew%, bevorzugt bei 0,001 bis 1,5 Gew%, besonders bevorzugt bei 0,002 bis 1 Gew%, bezogen auf das zu stabilisierende (Meth)acrylmonomer liegt.

3. Stabilisiertes (Meth)acrylatmonomer nach einem der vorhergehenden Ansprüche, **dadurch gekennzeichnet, dass** das (Meth)acrylatmonomer Hydroxyethylmethacrylat ist.

4. Stabilisiertes (Meth)acrylatmonomer nach einem der vorhergehenden Ansprüche, **dadurch gekennzeichnet, dass** das (Meth)acrylatmonomer eine Verbindung der Formel (3) ist 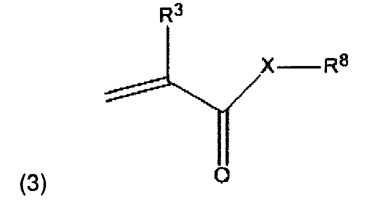 worin der Rest R³ Wasserstoff oder Methyl darstellt und -X- unabhängig 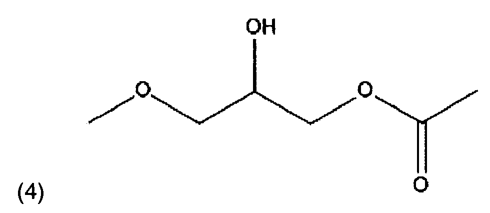 oder 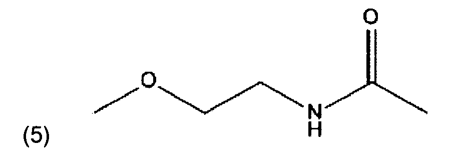 oder 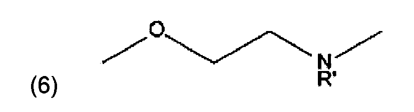 sein kann, worin R' Wasserstoff oder ein Rest mit 1 bis 6 Kohlenstoffatomen ist, und R⁸ einen linearen oder verzweigten Rest mit 8 bis 40, vorzugsweise 10 bis 30 und besonders bevorzugt 12 bis 20 Kohlenstoffatomen bedeutet, der mindestens eine C-C-Doppelbindung aufweist.

## Claims

1. Stabilized (meth)acrylic monomer, **characterized in that** it comprises, during synthesis, storage and transport, the compound of the formula (1) 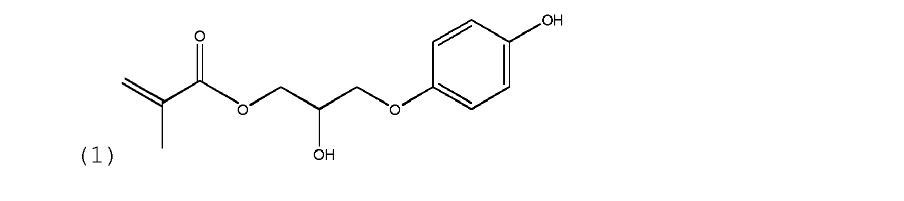 as a polymerization inhibitor.

2. Stabilized (meth)acrylic monomer according to Claim 1, **characterized in that** the concentration of (1) is 0.0001 to 2% by weight, preferably 0.001 to 1.5% by weight and more preferably 0.002 to 1% by weight, based on the (meth)acrylic monomer to be stabilized.

3. Stabilized (meth)acrylic monomer according to either of the preceding claims, **characterized in that** the (meth)acrylic monomer is hydroxyethyl methacrylate.

4. Stabilized (meth)acrylic monomer according to any of the preceding claims, **characterized in that** the (meth)acrylic monomer is a compound of the formula (3) 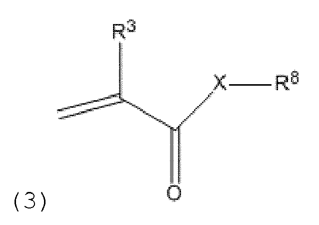 in which the R³ radical is hydrogen or methyl and -X-may independently be 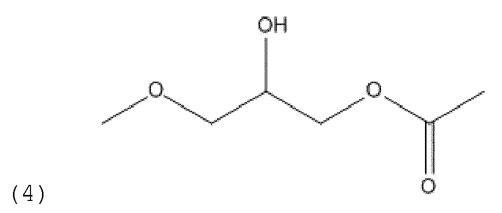 or 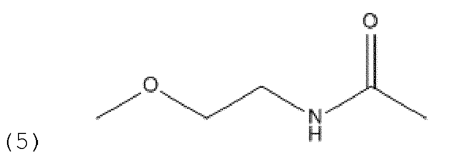 or 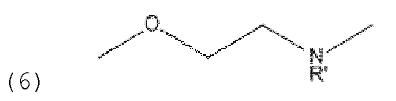 in which R' is hydrogen or a radical having 1 to 6 carbon atoms, and R⁸ is a linear or branched radical which has 8 to 40, preferably 10 to 30 and more preferably 12 to 20 carbon atoms and which has at least one C-C double bond.

## Revendications

1. Monomère (méth)acrylique stabilisé, **caractérisé en ce qu'**il contient, pendant la synthèse, le stockage et le transport, en tant qu'inhibiteur de polymérisation le composé de formule (1)

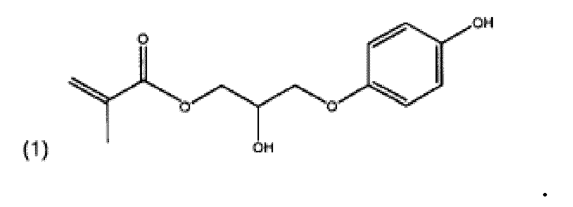

2. Monomère (méth)acrylique stabilisé selon la revendication 1, **caractérisé en ce que** la concentration de (1) vaut de 0,0001 à 2 % en poids, de préférence de 0,001 à 1,5 % en poids, de façon particulièrement préférée de 0,002 à 1 % en poids, par rapport au monomère (méth)acrylique à stabiliser.

3. Monomère (méth)acrylique stabilisé selon l'une quelconque des revendications précédentes, **caractérisé en ce que** le monomère (méth)acrylique est le méthacrylate d'hydroxyéthyle.

4. Monomère (méth)acrylique stabilisé selon l'une quelconque des revendications précédentes, **caractérisé en ce que** le monomère (méth)acrylique est un composé de formule (3) 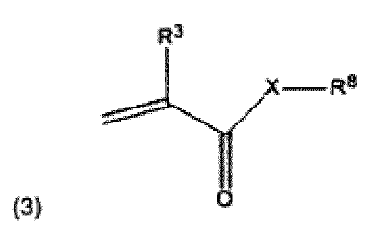 dans laquelle R³ représente un atome d'hydrogène ou le groupe méthyle et -X- peut être indépendamment 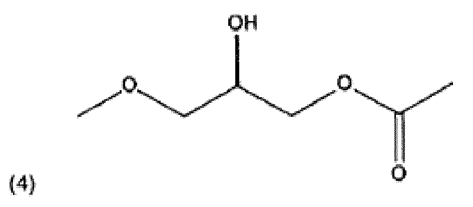 ou 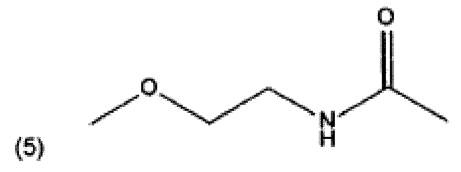 ou 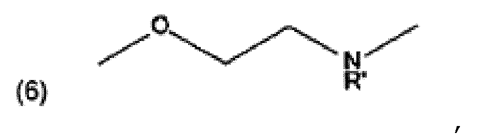 où R' est un atome d'hydrogène ou un radical ayant de 1 à 6 atomes de carbone, et R⁸ représente un radical linéaire ou ramifié ayant de 8 à 40, de préférence 10 à 30 et de façon particulièrement préférée 12 à 20 atomes de carbone, qui comporte au moins une double liaison C-C.
